# EUROPEAN PATENT SPECIFICATION

(11) **EP 0 493 484 B1**
(45) Date of publication and mention of the grant of the patent: **26.02.1997**
(21) Application number: 90914615.1
(22) Date of filing: 18.09.1990
(51) Int. Cl.: G01N 33/543, G01N 33/552, G01N 21/77

(54) **METHOD AND APPARATUS FOR DETECTION OF AN ANALYTE**
VERFAHREN UND VORRICHTUNG ZUM NACHWEIS EINES ANALYTEN
PROCEDE ET APPAREIL DE DETECTION D'UN ANALYTE

(30) Priority: 18.09.1989 US 408291
(43) Date of publication of application: 08.07.1992
(73) Proprietor: BIOSTAR MEDICAL PRODUCTS INCORPORATED, Boulder, CO 80301 (US)
(72) Inventor: MODDEL, Garret, R., Boulder, CO 80304 (US); MAUL, Diana, M., Denver, CO 80221 (US); ETTER, Jeffrey, B., Boulder, CO 80302 (US); STARZL, Timothy, W., Boulder, CO 80304 (US)
(74) Representative: Sexton, Jane Helen
(86) International application number: US9005317
(87) International publication number: WO9104491

(56) References cited:
- EP-A- 0 112 721
- EP-A- 0 276 968
- US-A- 4 876 208
- US-A- 4 886 761
- PATENT ABSTRACTS OF JAPAN, vol. 6, no. 195 (P-146), 05 October 1982/
- PATENT ABSTRACTS OF JAPAN, vol. 12, no. 412 (E-676), 31 October 1988/

## Description

### FIELD OF THE INVENTION

This invention relates to the field of biochemistry and chemistry, with applications in such areas as immunoassay, nucleic acid sequence analysis, clinical chemistry, enzyme assay, and the study of ligand/anti-ligand binding pairs such as toxin-receptor or hormone-receptor combinations, etc. This invention is a solid phase assay using direct physical detection methods based on optical interference properties. In a solid phase assay, the receptive material is immobilized on a coated support surface. The immobilized receptive material is exposed to a fluid that may contain the analyte, a molecule of interest capable of binding to or reacting with the receptive material. The analyte can be either organic or inorganic. In general, the roles of the receptive material and the analyte in the assay can be reversed.

### BACKGROUND OF INVENTION

Assays exist in the art for monitoring reactions between homologous couples such as antigen-antibody binding, hormone-receptor binding, enzyme assays, and nucleic acid hybridization. The critical part of assay design in each case is deciding how the signal will be generated. Colored reaction products, biological activity at the cellular level, and radioactive, fluorescent, or luminescent tags covalently bound to one of the reacting species have all been used. Two familiar examples in the field of immunoassay are enzyme-linked immunosorbent assay (ELISA) and radioimmunoassay (RIA). In all of these methods, the experimenter does not actually detect the binding of the two molecules, but some secondary activity related to the binding reaction. Most of these techniques require special steps for signal generation, adding to labor costs, and often require the use of hazardous materials and expensive instrumentation.

A more advantageous means of detecting binding or reaction between two species of a homologous couple is to detect a change in some physical property that is a direct result of binding or reaction. For example, if the receptive or reactive material is present as a thin film immobilized on a support, then any method that can measure a change in thickness, mass, optical mass or some other physical property of the thin film after binding or reaction with the analyte would be a means for direct physical detection. This invention provides a direct physical detection method, based on the principles of optical interference in thin films.

Many different techniques have been tried in the search for an accurate, rapid, inexpensive method of visually detecting an analyte of interest. Assays utilizing optical detection techniques have been researched and developed by Giaever. In U.S. Patent No. 3,926,564, issued December 16, 1975, Giaever describes a technique and device for visual detection of double layers of immunologically reactive biological material. Giaever teaches two devices: one is a substrate coated with metals, the second is a substrate coated with metals and semiconductor materials. A specific embodiment was fabricated by depositing an alloy of indium and gold on glass slides, and then heating the slides to generate oxidation of the indium. The device was then coated with a thin layer of biological material which caused little color change on the surface of the device. This bound biological material was then exposed to a second fluid with a biological material which was reactive to the first material. The binding reaction between the two biological materials produced a spot of a contrasting color on the device. Although quite ingenious, this method has not been proven to be totally adequate.

A second method of visual detection was expounded upon in two Japan patent applications Sho 61-222058 and Sho 61-222057, filed in September 22, 1986 and issued to Shiro & Kawaguchi; and in a European patent application publication no. EP 261642 by the same inventors filed on September 22, 1987. The method and device discloses a metal substrate or a glass substrate coated with a metal topped with a light interference layer coated with a biological compound capable of detecting a second biological compound. The European application also teaches sputtering a layer of colloidal gold on the reacted device to improve the contrast between the reacted and the unreacted portion of the device.

The method of making this invention produces some stability problems. This invention coats layers of material onto the device by such methods as the Langmuir-Blodgett technique, which may result in the layers delaminating. To avoid this problem in one embodiment, a coating of a reactive layer of receptive material is utilized to covalently bind the analyte to the receptive material. This doesn't solve the problem because the receptive material and bound analyte can detach because the receptive material is not covalently bound or adequately attached to the device. Furthermore, the visual contrast between a reacted portion of the device and unreacted portion of the device is not readily detectable without signal amplification techniques such as sputtering with colloidal gold.

A disadvantage inherent in the prior art devices shown by Giaever and Shiro & Kawaguchi is the inability to generate a strong visible color change when binding occurs. The reason the prior art has been unable to produce a device which is highly accurate and has a visible signal is because of the random selection of materials used to generate an interference effect. The device's background interference colors are not optimized to yield highly contrasting roll-over points. A rollover point is defined as the thickness of an anti-reflective layer or combination of layers at which the human eye perceives a substantial color or intensity change due to a relatively minor thickness change. Therefore, the accuracy and sensitivity of the device is limited. The strength of the generated signal varies with respect to the amount of analyte available for binding to the receptive material, and the number of active binding sites on the device. High concentrations of analyte are often necessary for signal generation; because low concentrations of analyte produce a relatively small change in the incident light pathway which does not produce a visible interference shift. Similarly, analytes which are small in size produce a nondiscernable signal and therefore cannot be easily detected by prior art devices. Even when a strong binding reaction occurs due to a high concentration of a small sized analyte the colored signal is often only faintly visible because of the randomly selected materials. The weakness of the signal has led to the use of amplification techniques to increase the visibility of the signal. For an example of amplification techniques see European patent application publication no. 261642 previously cited.

A further disadvantage of the prior art devices include the introduction of metals and their commensurate expense into the device. Metal substrates, or metal oxide layers disposed thereon, can be utilized but are less desirable because of their surface properties. The surface energy and the surface density of the binding locations change and therefore the binding effect or the absorption of materials cannot always be controlled within a reasonable degree of accuracy.

A third technique and device is disclosed in U.S. patent 4,558,012 issued December 10, 1985, to Nygren. This technique and device does not use metals in the device; however, the device is limited by its composition to only a few chemistries for linking the receptive material to the device.

EP-A-112721 describes an assay technique based on a diffraction grating. The technique comprises coating a preformed surface (grating) on a substrate with a thin film of a material capable of binding the species to be assayed, contacting the coated surface with the sample and observing the optical properties of the surface in order to determine a qualitative and/or quantitative change in optical properties as a result of the binding of the species onto the material.

EP-A-276968 describes a diffraction immunoassay in which a uniform layer of binding reagent is adhered to a smooth solid surface and the surface is exposed to UV radiation through a shadow mask with diffraction grating lines to selectively deactivate the binding reagent leaving a biological diffraction grating design of lines of active binding reagent. The solid surface is a polysilicon or single crystalline silicon surface.

Development of an inexpensive, selective device capable of easy activation and capable of producing a highly visible signal is essential to surmount the problems of the prior art.

It is therefore an objective of the present invention to provide a simple and sensitive assay based on visual detection of a visible interference shift produced by the reactions and binding of receptive material and the analyte of interest.

A further object of the present invention is to provide a device onto which a variety of concentrations of analyte can be detected.

The present invention includes novel compositions for the direct detection of the analyte, specific to the receptive material bound to the surface of the apparatus for the purpose of identification or quantification of the analyte. The present invention in its broadest sense provides a method of detection of an analyte comprising the steps of depositing one or more anti-reflective coatings onto a substrate material, affixing receptive material specific to the analyte of interest to the top layer of anti-reflective material; contacting the receptive material with a fluid to be tested for the corresponding analyte of interest, and then examining the reflection produced by the coated article to determine whether a change in reflection of the article has occurred.

According to the invention there is provided an analyte detecting article comprising a substrate supporting on its surface not less than one layer of anti-reflective material adhering thereto; the top layer of said anti-reflective material supporting on its surface a layer of analyte receiving material capable of analyte interaction; said at least one anti-reflective layer comprising an anti-reflective coating having a thickness of about one 1/4 the wavelength of light in the medium and a refractive index of abut the square root of the product of the indices of refraction of the medium directly above and below said layer.

According to the invention there is also provided the process of detecting the presence of the analyte that is performed by instrumentation, or by human vision, or with at least one filter or with at least one polarizer or at an angle other than normal.

### DESCRIPTION OF THE INVENTION

This invention is embodied in a device comprised of a substrate, one or more anti-reflective materials placed thereon, and a top anti-reflective layer capable of being activated to become the receptive material or to attach the receptive material by whatever mechanism to the device. The selected receptive material must be capable of binding and/or reacting with a chosen analyte. When a fluid sample containing the analyte of interest is exposed to the receptive material, the binding reaction between the material and analyte causes a change in the thickness, mass, or optical thickness on the device resulting in an optical interference color change.

### Step 1 Substrate Selection

The first element of this device is the substrate. This invention provides for a variety of diverse substrate materials and formats to suit the desires of the end user. The substrate is selected to have some of the various attributes described herein. The substrate can be formed of, or have coated on it, a diffusely or specularly reflective material such as polished silicon, chromium, etc.; or the substrate can be formed of a transmissive material such as certain plastics, glass, quartz, etc. The substrate may be a solid support, rigid, flexible or semi-flexible support, a pellicle or a gel. Examples of some suitable materials for making a substrate are quartz, plastic, silicon, non-metals, metals or functionally equivalent materials capable of having anti-reflective material coated onto the surface.

The refractive index and the optical thickness of the material chosen to act as the substrate is an important element in the design of this highly sensitive optical device. The material capable of acting as the substrate is subjected to only two restrictions: first it must be capable of being coated with an anti-reflective material, and second in the simplest case the refractive index of the substrate should be approximately equivalent to the square of the refractive index of the material directly above it or in a more complex device the refractive index of the substrate should be selected to generally fit one of the formulas in Table A (although a wide variety of ratios between the refractive indices of the substrate and the material directly above it can be utilized). The second criterion is important for signal generation. The interference colors produced by both the device and by the reacted portion of the device should be of visually contrasting colors or of such intensities to yield a highly visible signal. For example, use of a silicon wafer with a refractive index of approximately 4.1 (it varies with wavelength) allows this device to be designed with a wide variety of corresponding anti-reflective materials. Those skilled in the art will realize that various other substrate materials are equally suited for use in this device so long as they satisfy the above criteria. While a silicon wafter of approximately 12.7 cm (4 inches) in diameter and .05 cm (.02 inches) thick has been utilized with successful results the dimensions of the wafer are not critical to this invention.

### Step 2 Selection of the Anti-Reflective Material(s) and Affixation to the Substrate

After selection of the appropriate substrate an anti-reflective coating is selected and affixed to the substrate. The anti-reflective coatings are deposited onto the surface of the substrate by known coating techniques, for example, by sputtering or by vapor phase deposition in a vacuum chamber. Various other useful coating techniques are known to those skilled in the art. The deposition of anti-reflective coatings can be done so that anti-reflective material covers the entire substrate or so that a patterned or wedged layer of anti-reflective material is coated on the substrate. Materials useful as anti-reflective coatings may have some of these four attributes, namely (1) the material is generally clear and significantly transmissive at the thickness utilized, (2) stable at room temperature, (3) sufficiently stable to withstand the deposition techniques, and by definition (4) capable of suppressing some wavelength of reflective light when coated.

The anti-reflective material can also be an electro-optical material. Electro-optical materials or magneto-optic materials which are capable of changing this refractive index characteristic when subjected to magnetic or electrical fields can be utilized to enhance the precision of the rollover point. The electro-optical material should be selected so that the final refractive index which can be produced by the magnetic or electrical fields is approximately the refractive index necessary to produce the most precise rollover point. Anti-reflective material serves two functions, first, to produce an interference color, and second, to provide a top layer onto which receptive material can be affixed in such a manner as to maintain the receptive material's capacity to selectively adsorb or bind any analyte of interest present in the fluid to be contacted with the apparatus.

The anti-reflective material in this invention produces an interference color by destructive and constructive interference of certain wavelengths of light; for example, an anti-reflective layer of silicon dioxide coated at a thickness of 50 nm (500 Angstroms) on a silicon substrate produces a tan interference color. Silicon monoxide on a similar substrate and at a similar thickness produces a gold color, by suppressing blue wavelengths on the order of 480 nm.

The unique selection of the combination of a substrate and anti-reflective material to form a device with a precise roll-over point is based on the presence of an anti-reflective coating essentially having a thickness of approximately one-fourth of the wavelength of light in the medium, and a refractive index that approximates the square root of the product of the indices of refraction of the media immediately adjacent above and below it. Roll-over point is defined as the enhanced visual effect obtained by the measured thickness of one or more anti- reflective layers sufficient to permit the human eye to observe distinct and substantial changes in color or intensity which signals the presence of a detectable substance such as in an assay. Preferably this device will have few anti-reflective layers, but more complex devices have more anti-reflective layers which correspond to the formulas listed in Table A.

One characteristic of the coated anti-reflective material is the ratio of reflected/transmitted light. Whenever incident light passes from one medium into a second medium with a different refractive index, a portion of the light is reflected at the interface and a portion of the light is transmitted. To achieve the desired ratio of reflected/transmitted light the substrate is coated with thin layers of anti-reflective material. These anti-reflective layers act to cancel, by destructive interference, the reflections from the outer surface of the layer and the outer surface of the substrate. To achieve partial destructive interference the reflections must be of comparative intensities and approximately 180 degrees out of phase. Anti-reflective coatings with an optical thickness of lambda/4, when lambda is the chosen wavelength, produce reflections that will be out of phase.

To achieve a minimum reflectance of incident light lambda/4 should be equal to the optical thickness of the coated material. Optical thickness is defined as the physical thickness of the coating multiplied by the refractive index of that coating. Therefore, in the preferred embodiment of this invention the optical coatings are usually deposited with a thickness of about 1/4 wavelength optical thickness, or any odd number of 1/4 wavelengths of optical thickness, or in some combination of indices and thickness to achieve a minimum in the reflectance, as listed in Table A.

However, it is necessary not only to coat the correct thickness, but it is also necessary to have reflections of approximately equal intensity at the air/film and film/substrate interfaces. To establish equal intensities in the simplest case, the refractive indices should form a geometric progression. Since the refractive index of air is 1.0, the refractive index of the AR coating should be approximately equivalent to the square root of the substrate. In other words the greatest reduction in reflection occurs for an anti-reflective coating having a thickness of approximately one-fourth wavelength optical thickness of light in the medium, and a refractive index which is the square root of the product of the refractive indices of the media directly above and below it.

The anti-reflective materials will be approximately one-fourth wavelength optical thickness for one specific wavelength of light. The light used to irradiate the medium can be polychromatic or monochromatic depending on whether a color change or a change in intensity i.e. light-dark change is preferred. When determining the thickness of the anti-reflective coating the thin layer of receptive material coated to the top of the anti-reflective material or any other coated layers should be factored into the equation to yield the optimal thickness of the anti-reflective material.

A substrate such as the polished silicon wafer has a refractive index of approximately 4.1 for 540 nm light (green). To maximize the precision of the roll-over point of the device in accordance with the first equation of Table A, the anti-reflective material selected should have approximately 2.02 index of refraction. However, it has been found that materials having refractive indices close to the square root of the substrate can also be used with adequate results. The further the material's index of refraction is from the perfect square root of the substrate index of refraction, the less distinct the roll-over point becomes. The best results on silicon have been obtained with materials having refractive indices of 2, such as possessed by silicon nitride or 1.9-1.8 such as possessed by silicon monoxide. Other anti-reflective materials that have similar refractive index and could be utilized with this selected substrate include, but are not limited to: tin oxide, zinc oxide, chromium-oxide, barium titinate, cadmium sulfide, manganese-oxide, lead sulfide, zinc sulfide, zirconium oxide, nickel oxide, aluminum oxide, boron nitride, magnesium fluoride, iron-oxide, silicon oxynitride, boron oxide, etc.

A device with a highly sensitive and precise roll-over point can be developed with a variety of substrate materials with differing indices of refraction and with suitable corresponding anti-reflective materials alone or in combination. The number of anti-reflective layers coated onto the substrate can be any integer 1 to infinity. The number of layers is limited only by two factors: the indices of refraction of the selected materials should follow approximately an arithmetic scheme, and the cost involved in multilayer coating. It is expected that as coating technology advances and as contemplated within this invention, the multilayer practice can easily be utilized.

The slide apparatus when coated at a specific thickness with a silicon nitride anti-reflective layer suppresses certain wavelengths in the visible blue range and therefore reflects a brownish-gold interference color. Although a brownish-gold interference color is utilized in this combination of elements, the visual interference color of the slide can be any suitable color in the spectrum; depending on the material of the substrate selected, the chemical composition and refractive index of the anti-reflective layers selected, and the thickness and number of coated layers.

The optical thickness of the anti-reflective material can be determined by the equation n_{f} . t = lambda/4 if a specific wavelength is selected. This invention can be designed to utilize white light and all of its encompassing wavelengths. Therefore the chosen wavelength can be selected from a range of wavelengths, or the invention can be designed to utilize a selected monochromatic wavelength.

The anti-reflective material can be coated to approximately a quarter of a wavelength of visible light in thickness or alternatively an odd number of 1/4 wavelengths optical thickness can achieve similar results. As the thickness of the anti-reflective material increases the device more closely approximates the principles underlying the Fabry-Perot interferometer. Within the invention described the method for generating a device with a single anti-reflective layer is usually referred to; but it is also contemplated and disclosed that a device with a precise roll-over point can also be made with single or multi-layers of anti-reflective material by applying the following formulas listed in Table A (see Handbook of Optics, 8-48, 8-49, Driscoll, Vaughan [1978]).

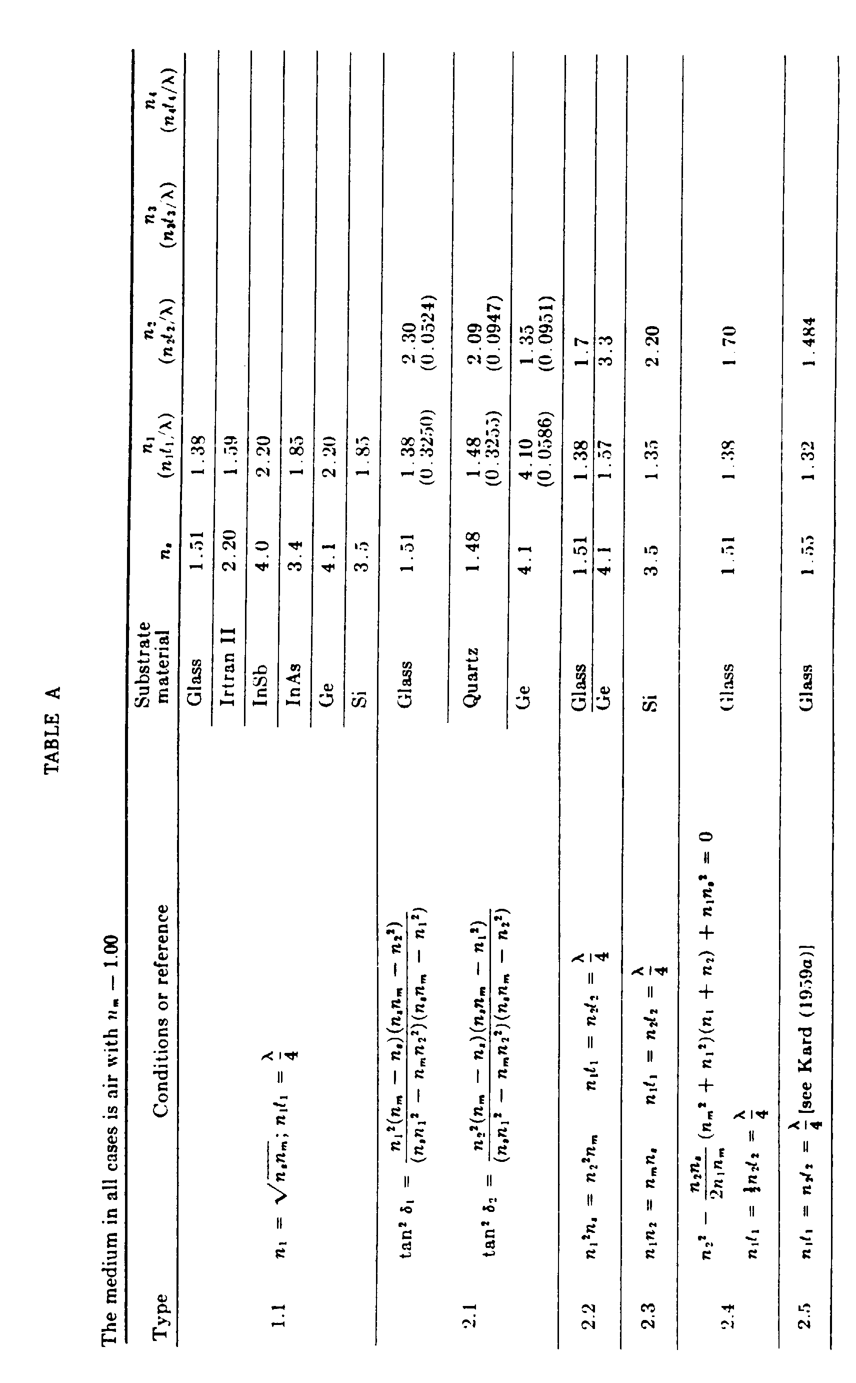

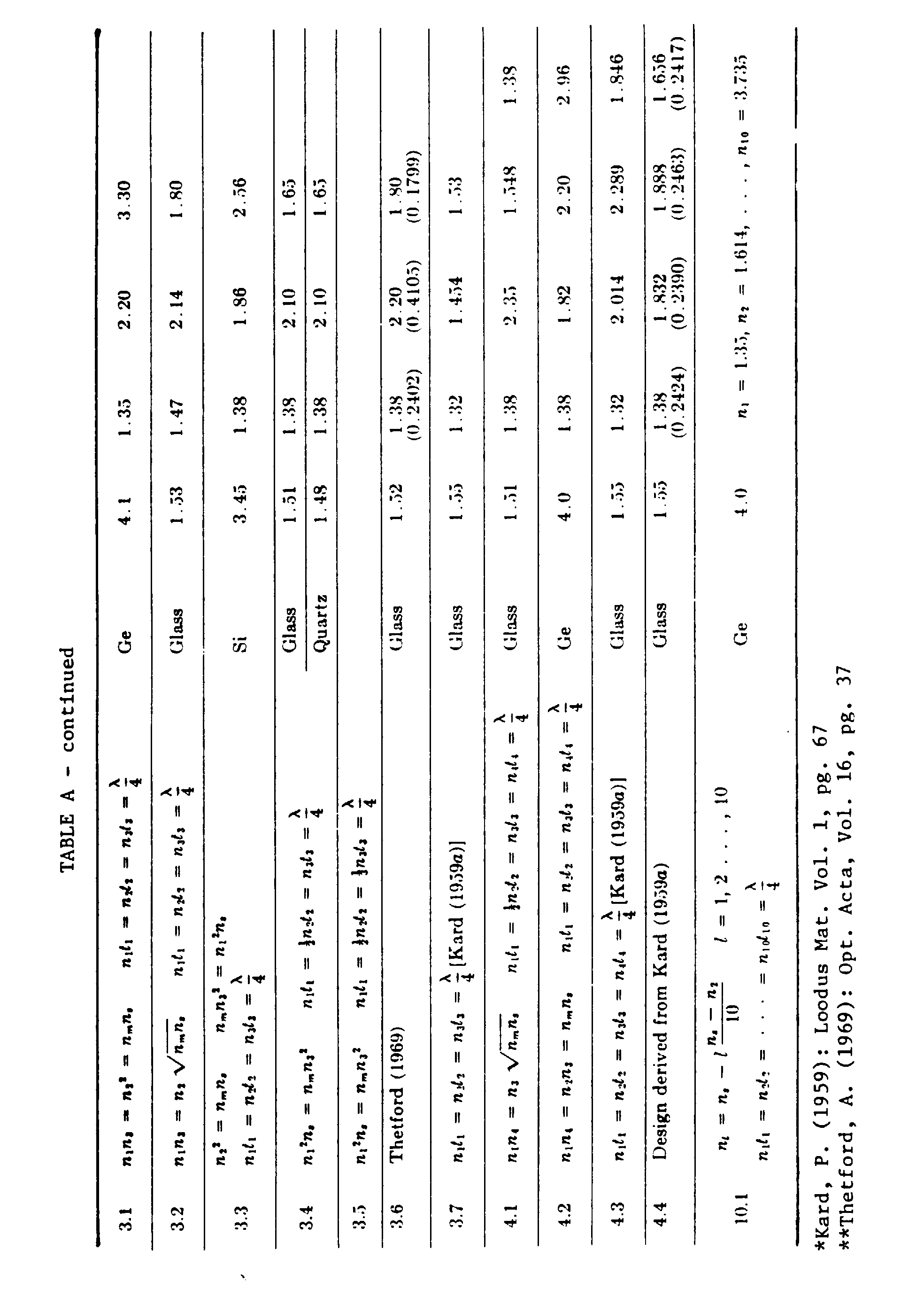

These formulas generate refractive-index combinations between the substrate, the anti-reflective layer(s) and the medium will yield zero or nearly zero reflectance at least at one wavelength. These refractive-index combinations are selected to maximize the change in the suppressed wavelength with a minimal change in thickness.

By utilizing Table A to design the stacking of the anti-reflective materials the device will generate a bright background interference color. Because the previous table is designed for normal incidence of light, the optimal device will be adjusted slightly to account for the 5 - 30 degree deflection off normal viewing angle at which most people view the device. (The device can be adjusted for whatever viewing angle is selected). By using a wedged model or by further calculation, a device with a precise roll-over point can be made. While it has been noted that the more accurate devices are usually in the first order color fringe; if the device is coated properly any order of color fringe can be employed. These design techniques can also be utilized to generate devices with signals or backgrounds in the ultraviolet or infrared region of the spectrum. These types of devices could require instrumented detection of the bound analyte.

The thickness of the coated anti-reflective material is dependent on the design selected, on the precision of the coating process utilized and on the characteristics of the selected material. When coating anti-reflective material outside influences usually cannot be totally controlled. As a result, visual selection of background interference color of the device after coating with anti-reflective material, can often be appropriate.

A model was developed to determine the proper background interference color. When the selected substrate is a silicon wafer and the anti-reflective material selected is silicon nitride, the most highly contrasting and distinctive roll-over point appears to exist when the colors observed are brownish-gold to magenta. A silicon wafer was prepared with a thick coating of silicon nitride so that the wafer appears to be a deep blue, the last color in the first order fringe. Then the anti-reflective material was etched off the wafer in a phosphoric acid bath into a wedged shape. The anti-reflective material was etched off so that 30 nm (300 Angstroms) remained at one end of the wedge and 70 nm (700 Angstroms) remained at the other end of the wedge. At 180°C the silicon nitride is etched off at approximately 2 nm (20 Angstroms) per minute. Suitable etching acids are commercially available for various anti-reflective materials.

The etched, wedged device shows visually different interference colors and differing roll-over points. The thickness of the anti-reflective material is then measured at the beginning of the roll-over point which appears to be the most distinctive visual change. This is the optimal anti-reflective thickness, because even small amounts of bound analyte will allow the reacted portion of the device to roll-over to the new interference color.

The device which is designed to be coated at the thickness selected by viewing the wedged model is coated with a layer of receptive material prior to completion. The optical thickness (the physical thickness multiplied by the refractive index) of the receptive material can be figured into the equation. The ratio of the refractive. index of the anti-reflective materials: to the top layer of material, and to the refractive index of the receptive material are multiplied by the physical thickness of the each individual material. This equation is then employed to determine the optimal thickness of the anti-reflective material. When a top layer is not utilized the ratio of the refractive index of the anti-reflective material and the receptive material can be multiplied by the physical thickness of the receptive material. The resultant amount can be etched from the anti-reflective material to maintain the optimal interference color. It should be understood that a wedged model showing the parameters of a specific device can be developed once and repeatedly utilized. Furthermore, although a physical model is often helpful, it is not essential to the development of a device with a highly distinct roll-over point.

The model for a silicon wafer coated with a silicon nitride that was chemically activated to attach 5 nm (50 Angstroms) of receptive material with a 1.5 refractive index revealed that approximately 55.3 nm (553 Angstroms) of anti-reflective material should be coated to the device. This amount of material is subject to variations due to changes in refractive index of the substrate or the coated material. At the selected thickness the device appeared to be a brownish golden color, after the selected receptive material was affixed to the surface, the interference color remained similar to the original brownish golden color, as was intended.

### Step 3 Activation

Prior to securing the receptive material to the device, the device was coated with a top layer of anti-reflective material capable of being activated to secure the receptive material to the device. This top layer is coated by a commercially known process in the same manner the anti-reflective material was coated to the device. However, where the original anti-reflective material coated onto the device is capable of being chemically activated to facilitate the covalent binding or adsorption of the receptive material to the device, no top layer is necessary. For example, a silicon nitride interference layer was activated without a coating of a top layer. In another embodiment, a top layer of silicon dioxide and various other materials were coated onto the silicon nitride at a thickness of approximately 0.1-10 nm (1-100 Angstroms). The thickness of the top layer varies widely but since a thick layer affects the interference effect by changing the path length of the incident light; when thicker layers were used this was taken into account in determining the thickness of the coating of anti-reflective material.

The material which is activated is most effective if it has the following three characteristics: easy to coat to the device, easy to chemically activate, and, when activated, capable of producing a surface of densely populated binding sites for the receptive material. These three characteristics have been found in a variety of silicon compounds, especially silicon nitride and silicon dioxide.

Any of the methods of binding receptive material, known to those skilled in the art, can be utilized within the scope of this invention.

Although utilizing materials which produce a highly visible roll-over point and thus a highly contrasting change in interference colors or intensities has lessened the need for massive binding of analyte to the device, it has not eliminated the need for a good population of binding sites. If there are numerous active binding sites available on the activated receptive material, then most of the analyte in the sample can react and bind to the device. This binding reaction changes the path length of the incident light and causes an interference color change.

Various top layers of material such as silicon oxynitride, silicon dioxide, and silicon monoxide along with various other silicon compounds have been activated by silane chemistry to generate functional groups capable of covalently bonding or to otherwise attaching various receptive materials or in some instances the analyte. These techniques rely on the derivatization of surface silanol groups with functionalized trialkoxysilanes. These materials and the respective chemistries produce sufficient binding sites to produce a highly visual interference color signal. It should be understood by those skilled in the art that a wide range of activation chemistries is useful depending on the material which is being activated. The materials can be activated to covalently bind, adsorb or by whatever mechanism adhere the receptive material to the device.

Prior to activating the surface, the device can be stripped of organic particles which can cause non-specific binding and generate background noise. One method of stripping the surface of the device is to oxygen plasma etch it in a vacuum chamber. The most appropriate method for cleaning the surface of the device is dependent on the characteristics of the anti-reflective materials and top layer selected. In some cases, the device need not be cleaned prior to attachment of the receptive material.

The apparatus is chemically activated to facilitate the attachment of the receptive material to the device. Adherence can be done by methods such as adsorption or covalent binding or by any other mechanism which will securely affix the receptive material to the activated coated substrate. Methods such as Langmuir-Blodgett can be employed, however the device made by this method is usually significantly less stable than other devices.

If the material activated is, for example, silicon oxynitride or silicon dioxide, then silane chemistry can activate the surface with functional groups which covalently bind the receptive material to the surface. An example of one method of activation follows: 25 coated wafers are placed within a vacuum dessicator containing approximately 2.5 microliters of N-(2-aminoethyl)-3-aminopropyltrimethoxysilane having a boiling point of 140 degrees at 15 Torr. The boiling point of the chosen aminosilane compound determines the optimal pressure and temperature for running the vapor phase deposition.

The vacuum dessicator is evacuated to 0.06 Torr for 30 minutes; and then the temperature is raised to 100 degrees over the course of one hour. The process coats approximately 2 to 3 nm (20 to 30 Angstroms) of active stable material onto the device.

### Step 4 Attachment of Receptive Material

Once the device is chemically activated, the attachment of the receptive material is performed by standard procedures. The material often should be diluted and buffered prior to adherence to the device. The steps necessary to attach the receptive material to the device will depend on the activation chemistry selected and the receptive material selected.

An example of one method of attachment of a receptive material to the activated device follows: The selected receptive material is human IgG solution which is placed in a plastic cell culture box containing the activated device. The human IgG solution consisted of 20 milliliters of ten mM phosphate buffered (0.9%) saline solution (PBS) at a pH of 7.4 and 20 micrograms per ml of human IgG (this provides an excess of human IgG), and a ratio of 1% by volume of glutaraldehyde.

This solution is allowed to incubate at room temperature in an agitation bath overnight. Following the incubation, the device is rinsed with distilled deionized water; next a blocking step can be implemented to decrease the amount of non-specific binding. Following this optional step the slide is rinsed and dried under a stream of N₂, or pressurized air, or allowed to air dry.

At this point the device is completely formed. When properly packaged and stored at 4 degrees C the device will be able to detect the analyte of interest for at least two years.

### Step 5 Assay for Analyte of Interest

The sample was next contacted with the device to determine if the sample contains the analyte. Prior to contacting the sample with the device it may be appropriate to dilute the sample with a diluent such as phosphate buffered saline. The ratio of sample to diluent and the diluent selected vary depending upon the nature of the sample and the assay technique selected.

Small analytes can be readily detected due to the design of the materials to produce a brilliant distinct roll-over point. However, it was desirable to bind small analytes to larger molecules that when bound to the receptive material produced a greater change in the path length of the incident light. This increased the signal generated by the reacted device. The analyte sample when bound to a larger molecule was then treated the same as any other analyte sample.

As will be understood by those skilled in the art, the sample can be contacted with the device in a variety of methods including, but not limited to, smearing on or spotting onto the device or dipping the device into the sample. Adherence of the analyte onto the receptive material can sometimes be enhanced by various methods including incubating the slide on a heating block, or placing the device under a heat lamp, or infrared lamp, or by allowing the device to incubate at room temperature until excess sample moisture has evaporated. It should be understood that techniques for enhancement of adherence of the analyte is a function of the receptive material and analyte selected.

Following the incubation the device is fully rinsed to remove any material not adhered to the surface, and then the device is dried by a stream of N₂, pressurized air blotting, with an absorbent pad or any other equivalent method.

### Step 6 Assay Detection Techniques

Two assay detection techniques, visual and instrumental, are primarily used for the detection of the bound analyte, although any suitable means of detection can be employed. For the examples described hereinafter, an interference color phenomena was used to visually ascertain, and an ellipsometric measurement of the change of thickness was used to instrumentally ascertain whether the fluid contained the analyte of interest.

The device embodying this invention can be irradiated with a polychromatic or monochromatic light source in the ultraviolet, visible or infrared regions of the spectrum to determine whether analyte has bound to the surface. The unreacted device formed with a silicon wafer and a silicon monoxide anti-reflective layer and a silicon dioxide top layer and a biological receptive material under polychromatic light reflects a gold interference color with an absorbance maxima of approximately 476 nanometers. In comparison, the reacted article under polychromatic light reflects (the gold color where no analyte is bound) a purple or a blue interference color with an absorbance maxima between 500 nanometers and 650 nanometers where the analyte is bound.

The color of the reacted spot depends directly on the concentration of the analyte bound to the surface. The higher the concentration, the more intense the color change or intensity appears.

When the analyte interacts to the receptive material the physical thickness of the material on the top of the substrate either increases or decreases. This thickness change results in a change in the optical pathlength; the new optical pathlength causes the interference color or intensity to change. In order to achieve the correct phase shift for destructive interference of a given wavelength in this invention, the thickness of the AR (anti-reflective) film coating, is usually slightly less than an odd number of quarter wavelengths of optical thickness. Because air is a less dense medium than the AR coats or the substrate, there is a *pi* phase shift for reflections at both interfaces. The identical phase shifts cancel each other.

Therefore, to determine the net phase shift the only factor is the optical path difference, which is defined as 2d x n_{f}. The definition of d is the actual measurement of the AR thickness, and n_{f} is defined as the AR film's refractive index. The actual phase shift is therefore 2dn/lambda multiplied by 2pi if in radians, or 360 if in degrees. The thickness of the AR is approximately some odd quarter wavelength optical thickness; in this example the actual optical thickness is approximately equivalent to lambda/4, lambda being the wavelength selected for peak performance. Therefore, the phase shift is about 180 degrees and there is partial destructive interference which reveals (in this selected example) a gold interference color because certain wavelengths are suppressed.

When the analyte binds to the receptive material on top of the device, the optical path is either increased or decreased as previously stated. In this example, the actual thickness change incurred by the binding reaction between the analyte and the receptive material resulted in an increase in material of approximately 2 nm (20 Angstroms). The amount of increase or decrease of material is a function of the size and amount of the analyte available and the amount of available reactive binding sites. Increases of 3 nm (30 Angstroms) of material are not uncommon. This change in the optical path yields a suppression of different wavelengths, thus resulting in a blue or purple interference color defining the area of binding of the analyte.

The clarity of the assay's interference signal is slightly dependent on the angle at which the assay is viewed. Therefore, the calculation of the materials and the thickness and the refractive indices which will produce the most optimized rollover point must take into account the viewing angle utilized. Although signal enhancement techniques are not necessary; it has been noted that various filters and polarizers can be used to further enhance the clarity of the interference signal at angles which are not normal to the surface of the assay.

To avoid the need for signal enhancement techniques the background interference color should be optimized to be near the roll-over point to yield a contrasting interference color or intensity when reacted. This provides a color contrast to which the human eye is highly sensitive.

Known concentrations of analyte reacted on a device can be read on the ellipsometer and the resultant data can be used to generate a standard curve for more quantitative results. It should be apparent that other methods and instruments of measurement of the analyte concentration can be employed within the scope of the present invention.

### EXAMPLE 1

### Detection of Carcinoembryonic Antigen (CEA) (Immunoassay - Non-Competitive Format)

A silicon wafter with a highly polished surface and refractive index of 4.09 at a given wavelength was coated with an anti-reflective material, silicon oxynitride, to a thickness of 65.3 nm (653 Angstroms) of material with an average refractive index of 2.0. To achieve the most distinct roll-over point, wavelengths of approximately 480-470 nanometers were suppressed. To suppress these wavelengths the thickness of the anti-reflective material had to be etched down to between 60 and 58.6 nm (600 Angstroms and 586 Angstroms) of material.

The optimal thickness of the anti-reflective material is further defined by the index of refraction and the thickness of the receptive material. This is often a negligible factor and can be disregarded, but in this instance it was determined that approximately 3 to 5 nm (30 to 50 Angstroms) of material having a refractive index of approximately 1.5 was to be coated to the top of the activated anti-reflective material. The ratio of the two indices of refraction multiplied by the coated thickness of the receptive material revealed that approximately 3.5 nm (35 Angstroms) of anti-reflective material should be etched off to achieve a more distinct roll-over point. Therefore, the slide was etched down to approximately 56-56.5 nm (560-565 Angstroms) of anti-reflective material. Then the device was chemically activated by application of N-(2-aminoethyl)-3-aminopropyltrimethoxysilane by the following procedure:
1. The coated wafer was oxygen plasma etched for 5 minutes in a .70 Torr oxygen atmosphere with a plate current of 175 D.C. milliamperes and 250 RF watts.
2. Immediately upon removal from the oxygen plasma etcher the wafer was placed in a quartz rack which was inserted into a vacuum dessicator with an attached vessel into which 2.5 microliters of the aminosilane was placed.
3. The vacuum dessicator was evacuated to 0.06 Torr for 30 minutes. Then the temperature of the dessicator was raised to 100 degrees C over the course of one hour. This process activated the wafer by the vapor deposition of aminosilane onto the wafer surface.
4. The wafer was now prepared for the adherence of the receptive materials. First, a solution comprised of 20 ml PBS, 0.1% glutaraldehyde by volume, and 150 microliters of IGAP which is a synthetic polypeptide that covers the active region of protein A was made up. (Other experiments substituted protein A or protein G for the IGAP and similar results were achieved.)
5. 200 microliters of monoclonal antibody (polyclonal can be substituted) specific for carcinoembryonic antigen was pipetted into the IGAP solution at 1 mg/per ml.
6. The combined solution was placed on top of the wafer in a petri dish and was incubated at room temperature in a shaker bath for two hours. When removed the petri dish was transferred to a cooling unit and allowed to incubate for 48 hours at 4 degrees C to further the adherence of the receptive antibody material. To remove all unbound material the wafer was rinsed with distilled deionized water and dried in a stream of N₂.

The golden tan device was then used to determine the presence of CEA antigen in five serum samples with known CEA concentrations.

An assay as described hereinafter, using the coated wafer, prepared as just described, yielded the data in Table 1 by the following procedures:
1. 10 microliters of all five samples of serum were contacted with the surface of the wafer, and allowed to incubate at 37 degrees C for seven minutes. In other experiments incubation at 45 degrees C for three minutes produced similar results.
2. After seven minutes the wafer was rinsed in deionized distilled water to remove all unbound material. Then the wafer was dried under a stream of nitrogen. Visual inspection of the slide revealed small pinkish spots with color intensities which correlated with the increasing analyte concentration levels.
3. An instrument was used to verify the increase of thickness produced by the binding reaction on the surface of the wafer. The change in the thickness of the reacted portion of the wafer was measured in gray scale units by a Sagax_{R} comparison ellipsometer.

**TABLE 1**

| Measurement of Bound CEA Antigen | | | |
|---|---|---|---|
| CONCENTRATION (ng/ml) | Average Gray Scale | Standard Deviation | CV |
| 0.0 | 20.1 | 2.73 | 13.6% |
| 2.0 | 31.67 | 1.86 | 5.7% |
| 4.0 | 36.94 | 1.86 | 5.0% |
| 8.0 | 44.68 | 2.56 | 5.6% |
| 16.0 | 60.85 | 3.73 | 6.1% |

### EXAMPLE 2

### Comparison of Slide Design (Comparison of Clinical Chemistry Assays)

Two silicon wafers were prepared with two different anti-reflective materials, to suppress approximately the same wavelengths of light. The first silicon wafer was commercially coated with silicon oxynitride and then etched to a thickness of approximately 59 nm (590 Angstroms). The second silicon wafer (refractive index of 4.1) was commercially coated with silicon dioxide having a refractive index of 1.46 and then etched to a thickness of approximately 80.8 nm (808 Angstroms) of thickness. The selected thickness of both anti-reflective materials suppress wavelengths of approximately 470-474 nanometer range. The first device was designed to yield a highly visible roll-over point; the refractive index of the silicon wafer is approximately 4.1 and the refractive index of the silicon oxynitride layer is 2.0, no receptive material was taken into account. The second device was designed to suppress the same wavelengths as the first device, however this device was not designed in accordance with the first equation in Table A and therefore did not yield a highly contrasting roll-over point. The same test was performed on each device.

The devices were activated by application of N-(2-aminoethyl)-3-aminopropyltrimethoxysilane by the following procedure:
1. The coated wafer was oxygen plasma etched for 5 minutes in a .70 Torr oxygen atmosphere with a plate current of 175 D.C. milliamperes and 250 RF watts.
2. Immediately upon removal from the oxygen plasma etcher, the wafer was placed in a quartz rack which was inserted into a vacuum dessicator with an attached vessel into which 2.5 microliters of the aminosilane was placed.
3. The vacuum dessicator was evacuated to 0.06 Torr for 30 minutes. Then the temperature of the dessicator was raised to 100 degrees over the course of one hour. This process activated the wafers by the vapor deposition of the aminosilane onto the wafer surface.
4. The wafers were activated, and the functional groups coated in the activation process acted as the receptive material and no separate material receptive was coated to the surface.

The first wafer with silicon oxynitride reflected a golden brown interference color; the second wafer coated with silicon dioxide reflected a brownish grey interference color. The two wafers were then used to determine human pregnancy. Four positive urine samples and one negative control sample were used.

The assay was performed as hereinafter described, using the first and second coated wafer. These assays yielded the data in Table 2.
1. The urine samples were diluted 1:1 in a 8.0 pH buffered solution of 0.02 M TRIS-HCl, and 0.7% bovine serum albumin, both commercially available from Sigma.
2. The five diluted samples (approximately 15 microliters) were then spotted onto the prepared wafers and allowed to incubate for 30 minutes at room temperature.
3. The wafers were rinsed in deionized, distilled water to remove all unbound materials, and dried under a stream of nitrogen.
4. Visual inspection of the first device (silicon nitride) revealed four highly visible purplish blue spots where the positive urine was placed; the negative urine showed no change in the wafer's golden brown surface color. Visual inspection of the second device (silicon dioxide) revealed four faintly visible grey blue spots where positive urine was contacted, and the negative sample showed no change in the brownish gray interference color.
5. The change in the thickness of the reacted portion of the wafers were measured in gray scale units by a Sagax_{R} Comparison Ellipsometer.

**TABLE 2**

| Urine Samples | Average Gray Scale Value | | Average Background | |
|---|---|---|---|---|
| | Dev #1 | Dev #2 | Dev #1 | Dev #2 |
| Pregnancy Pos. 1 | 65.1 | 68.1 | 41.0 | 46.0 |
| Pregnancy Pos. 2 | 57.1 | 62.8 | 41.0 | 46.0 |
| Pregnancy Pos. 3 | 49.9 | 58.0 | 41.0 | 46.0 |
| Pregnancy Pos. 4 | 55.2 | 61.4 | 41.0 | 46.0 |
| Pregnancy Neg. 5 | 43.0 | 48.0 | 41.0 | 46.0 |

Although the two wafers bound similar amounts of analyte to their respective surfaces, the visibility of the signal generated by the binding of the analyte to the devices was not similar.

The silicon oxynitride coated device revealed a highly contrasting vibrant bluish purple spot against a golden background. The silicon dioxide coated device revealed a washed out grey-blue spot against a grey-brown background. It is noteworthy that sample #3 which appears to have less analyte was barely discernible on the silicon dioxide device but highly visible on the silicon nitride device.

### EXAMPLE 3

### Wedged Detection of Rheumatoid Factor (Semi-Quantitative Assay)

A glass microscope slide support was coated with a chromium substrate by a commercially available coating process. The substrate was then coated with an anti-reflective material, cadmium sulfide, to a thickness of 50 nm (500 Angstroms) of material. The device was then mechanically etched in a dilute HCl acid to form a wedge of cadmium sulfide ranging from 0 to 50 nm (0 Angstroms to 500 Angstroms). The etched device was then coated with 5 nm (50 Angstroms) of silicon dioxide by a commercially available vapor deposition process. The wedged slide reflected a silver color from approximately 0 to 15 nm (0 to 150 Angstroms) of cadmium sulfide, tan from 15 to 25 nm (150 to 250 Angstroms), gold from 25 to 32.5 nm (250 to 325 Angstroms), brown-gold from 32.5 to 37 nm (325 to 370 Angstroms), magenta from 37 to 40 nm (370 to 400 Angstroms), deep purple from 40 to 44 nm (400 to 440 Angstroms), deep blue from 44 to 46.5 nm (440 to 465 Angstroms), a royal blue from 47.5 to 50 nm (475 to 500 Angstroms), when viewed under polychromatic light at a 45 degree angle from normal incident light.

This device was then chemically activated by application of N-(2-aminoethyl)-3-aminopropyl trimethoxysilane by the following procedure:
1. The coated wafer was oxygen plasma etched for five minutes in a vacuum at .7 Torr, with an oxygen atmosphere and a plate current of 175 D.C. milliamperes and 250 RF watts.
2. Immediately upon removal the coated wafers were placed in a quartz rack and inserted into a vacuum dessicator with a vessel containing 5 microliters of N-(2-aminoethyl)-3-aminopropyltrimethoxysilane. The vacuum was evacuated to C.06 Torr for 30 minutes. Then the temperature of the dessicator was raised to 100 degrees over the course of one hour to complete the vapor phase deposition of aminosilane.
3. 200 micrograms/per ml of BGG and 20 mls of PBS (previously described) and 1% by volume of glutaraldehyde were combined to form the receptive material solution. The wafers were placed in a petri dish and the receptive material solution was added.
4. To enhance the adherence of the receptive material the wafers were allowed to incubate at room temperature in an agitation bath for 15 hours.
5. Following incubation the unbound bovine gamma globulin proteins were removed from the wafers, by rinsing with distilled deionized water.
6. Following the rinsing of the slide apparatus to decrease the amount of non-specific binding, the apparatus was placed in a blocking solution and allowed to incubate for one hour in an agitated bath. The blocking solution was made of 2 ug/ml of acid hydrolyzed casein plus a ratio of 1% glycerol volume to volume and 2% sucrose weight to volume in PBS sufficient to bring the total volume to 20 mls.
7. Following the blocking process all excess blocker was removed by rinsing with deionized distilled water. Then the slide was dried under a stream of nitrogen.
8. The wedged slide was then smeared with two positive coated serum samples and a patient serum. One positive coated sample was a concentration of 700 IU per ml of diluent; the second positive control sample was a concentration of 175 IU per ml of diluent. These samples were prepared by the following procedure:
   a. The positive patient serum sample was diluted by adding PBS (previously described) at a 1:1 ratio (volume serum: volume diluent). One control positive serum sample was diluted by adding PBS at a 1:1 ratio to yield a concentration of 700 IU per ml. The 175 IU per ml control was obtained by a serial dilution of the 700 IU per ml sample. The IU per ml of the control samples can be varied according to the level of concentration usually found in a positive unknown sample.
   b. 5 microliters of each diluted sample or control was placed on each wafer; and the wafers was allowed to incubate on a heating block at 45 degrees for three minutes to enhance analyte adherence.
   c. The wafers were then rinsed with deionized water to remove all unbound material, then dried with a stream of pressurized air.

Upon visual inspection of the wedged device under polychromatic light the reacted controls on the slide reflected similar colors but had varying intensities. The 175 IU per/ml was a less brilliant color than the 700 IU per/ml was at the same anti-reflective thickness. The 700 IU/per ml, the high positive control, showed a visible signal on the silver background portion of the slide. The low positive 175 IU/per ml showed a visible signal at a thicker anti-reflective layer. The visible signal appeared on the tan to gold background portion of the slide. The most distinct color generated was at the roll-over point where the purplish-brown-gold background yielded a royal blue reaction signal.

The patient serum sample generates a smear which visually appears to be identical to the 175 IU/ml control. When visually compared to blue signal generated at the roll-over point appears to be of the same intensity. This semi-quantitative test format allows the patient's sample to be compared to the standardized color curves produced by the control samples coated on the device to determine the approximate amount of autoimmune indicator in the sample.

### EXAMPLE 4

### Detection of Serotonin/Hapten (Competitive Format)

A silicon wafer with a slightly irregular surface and an index of 4.02 at a specified wavelength can be coated with an anti-reflective material silicon oxynitride. The silicon oxynitride layer can be coated in accordance with the formula for a single layer of anti-reflection material. The refractive index of this coating of silicon oxynitride will be 1.92 and the coated thickness can be approximately 60.5 nm (605 Angstroms) [+/-0.5 nm (5 Angstroms)]. At this thickness the coated wafter produced a purplish-yellow interference color. The optimal coated thickness of this anti-reflective material should not be significantly changed by the minute amount of receptive material that is coated over the anti-reflective layers.

The device was chemically activated by application of N-(2-aminoethyl)-3-aminopropyltrimethoxysilane by the following procedure:
1. The coated wafer can be oxygen plasma etched for 5 minutes in a .71 Torr oxygen atmosphere with a plate current of 175 D.C. milliamperes and 250 RF watts.
2. Immediately upon removal from the oxygen plasma etcher the wafer can be placed in a quartz rack which will be inserted into a vacuum dessicator with an attached vessel into which 5 microliters of the aminosilane will be placed.
3. The vacuum dessicator should be run at .06 Torr for 30 minutes. Then the temperature of the dessicator should be raised to 100 degrees over the course of one hour. This process activates the wafer by the vapor deposition of the aminosilane onto the wafer surface.

If the above procedure is performed, the result should be a wafer with a derivatized amine surface. Due to the limited number of surface reactive sites for the aminosilane, it has been found useful to graft organic polymers to the surface amines to increase the density of reactive sites as well as to create a physical barrier between the material to be immobilized and the inorganic wafer surface. A convenient approach along these lines employs the polysaccharide dextran, which has been partially oxidized with sodium periodate. A buffered solution of oxidized dextran when reacted with the amine derivatized surface forms Schiff bases between the available aldehydes and amines. Reduction of these wafers with sodium borohydride should then result in a stable, high density polymeric alcohol surface.

Beginning with the polymeric alcohol surface, a number of techniques can be employed to activate the surface toward coupling with serotonin. Two distinct approaches to serotonin immobilization are:
1) A variable approach that will allow immobilization via nucleophilic displacement with either the phenol or the primary amine depending on the pH of the reaction medium.
2) A diazonium salt immobilization via electrophilic aromatic substitution of the phenol ring.

The basis of the first approach lies in the inherent reactivity of serotonin at various pH levels. All of these techniques rely on the nucleophilic displacement by serotonin of a reactive component on the surface. Theoretically, at low pH levels, the phenolic-OH will be deprotonated leading to immobilization through formation of an ether linkage with the phenoxide. At higher pH levels, near neutrality, the primary amine will be more nucleophilic than the phenol, leading to immobilization via secondary amine formation.

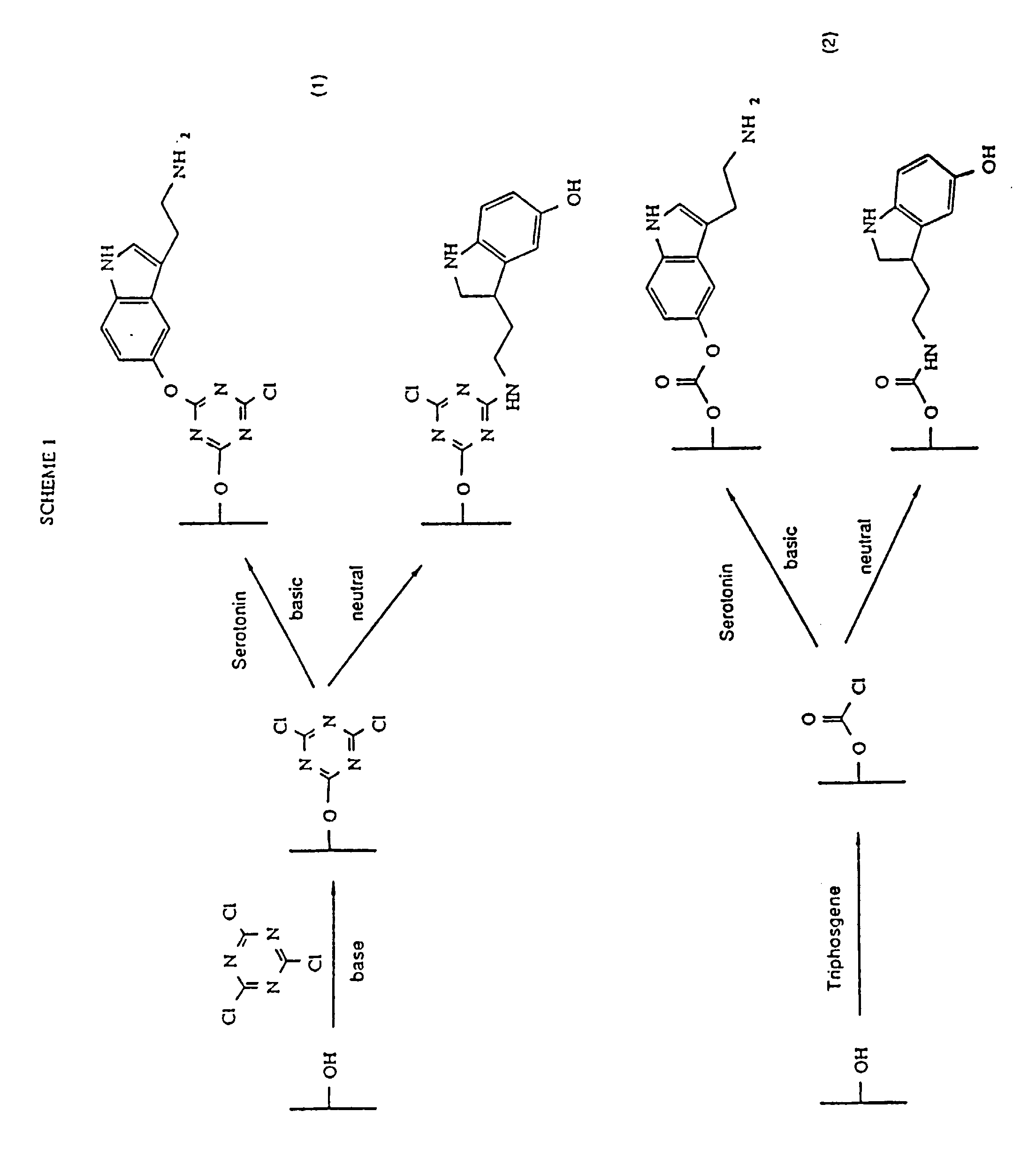

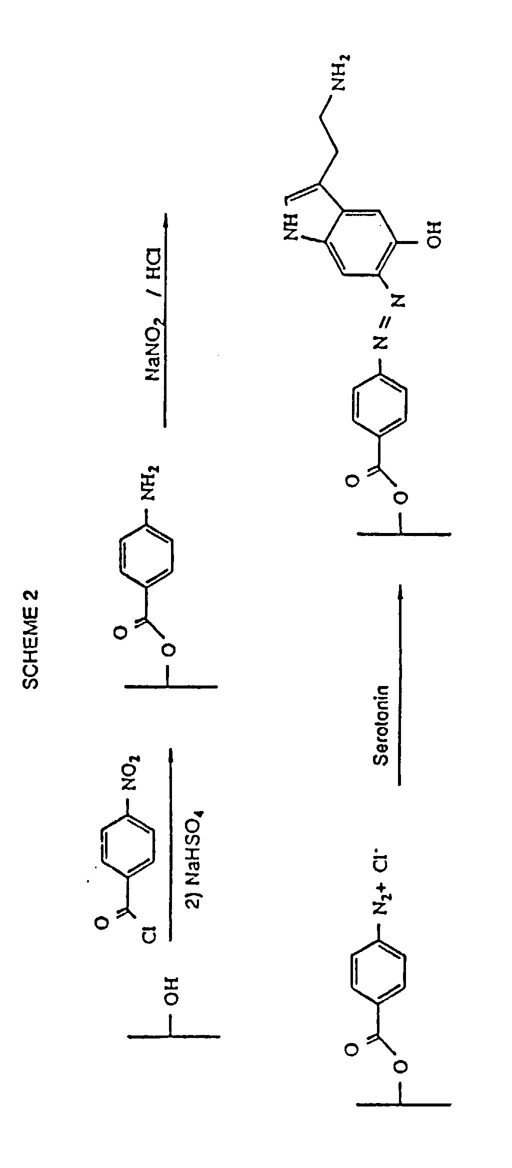

Two activation procedures should be amenable to this approach:
1) Cyanuric chloride activation
   Reaction of the polyalcohol surface with cyanuric chloride under basic conditions results in a dichloroalkoxytriazine that may then be reacted with either the phenol or amine as described above (see Scheme 1, equation 1). Cyanuric chloride is relatively easy to manipulate while maintaining high and specific reactivity. Due to the rigidity of the aromatic system, this linker tends to hold the immobilized material away from the surface.
2) Triphosgene activation
   Reaction of the polyalcohol surface with triphosgene, a synthetic equivalent of phosgene which is a solid and thus easier to handle, leads to chloroformate formation (see Scheme 1, equation 2). Phosgene activated alcohol surfaces possess high reactivity, however, the difficulty of handling phosgene itself has precluded significant utility of this process. This system may then be used to immobilize serotonin from either end as decribed above.

Immobilization via diazonium salt formation is the second approach. An activation procedure amenable to the second approach should be the reaction of the polyalcohol surface with 4-nitrobenzoyl chloride followed by immediate reduction with sodium hydrosulfite which would result in a 4-aminobenzoic ester derivatized surface. Reaction of this surface with sodium nitrite in HCl generates the unstable diazonium salt which when reacted with serotonin should result in an electrophilic aromatic substitution of the phenol ring (see Scheme 2). This technique will maintain the rigidity of the cyanuric chloride system, and not tie up either the phenol or the primary amine.

Wafers that have been coated with serotonin will then be exposed to various antibodies, both polyclonal and monoclonal, at different levels of concentration to observe the amount of binding that occurs.

Theoretically, the golden tan device could then be used in a competitive assay format to determine the presence of the hapten, serotonin, in the analyte solution. The analyte solution will be mixed with an antibody solution allowing a binding reaction to occur in solution. This solution should then be contacted with the hapten coated wafer. If there is a large amount of analyte (hapten) in the solution then there should be only a few antibodies that have not already bound to the hapten in solution. The unbound antibodies should bind to the immobilized hapten on the wafer surface, while the reacted hapten-antibodies should not attach to the surface. Therefore, an analyte solution with a large amount of analyte will generate little or no signal. If there is no analyte (hapten) in the solution, the antibodies in the solution will remain active and will bind to the surface of the wafer theoretically producing a bright royal blue interference color. The higher the analyte concentration the lower the royal blue signal should appear to the viewer. This test can be a semi-quantitative test. A wafer is tested with varying hapten concentrations and the color of the signals generated are reproduced on a paper strip. The colors on the paper strip are then compared to the color produced on a reacted wafer by the unknown analyte solution. An alternative semi-quantitative method is to supply each test kit with a wafer with the reacted hapten concentrations pre-marked on the slide. This alternative avoids any coloration errors in the printing, but can be more costly than the paper strips.

Although this is would usually be a visual non-instrumented test, a comparison ellipsometer can be used to verify the changes in film thickness due to the binding reactions. The optical thickness of the background portion of the wafer and the reacted portion of the wafer would be measured and the change in thickness compared. The measurements used to read thickness change are gray scale units. These units are digital representations of the light intensity that reaches the optical detector from the comparison ellipsometer. The value of the gray scale units can be related to analyte concentration to generate a standard curve. The data in Table 3 was generated by the detection of a different hapten on a similar test format; other than serotonin. It is projected that comparable instrumented data as found in Table 3 would be generated by the detection of serotonin. The assay would be run by the protocol given below:
1. Solutions with varying hapten concentration should be prepared. The first solution contained 5 microliters of PBS. The second solution should contain 500 ng hapten per milliliter of water. Serial dilutions should be used to produce 31 pg/ml water, 62 pg/ml, 125 pg/ml, 500 pg/ml, 1000 pg/ml and 10,000 pg/ml concentrations of haptens. These dilutions can be used to generate a standard curve.
2. A polyclonal antibody solution should be prepared with approximately 0.6 mg of antibody per ml of PBS.
3. Five microliters of the polyclonal antibody solution should be placed in 7 separate test tubes. Five microliters of the first PBS solution then should be added to one test tube and the solution then should be mixed. Five microliters of the serial dilutions of hapten will then be added to separate test tubes containing the antibody mixture and mixed.
4. Five microliters of solution drawn from each test tube should be contacted with the coated wafer. The wafer then should be allowed to incubate at 45 degrees C for three minutes.
5. After 3 minutes the wafer can be rinsed in deionized distilled water to remove all unbound material. Then the wafer should be dried under a stream of nitrogen. Visual inspection of the slide theoretically should reveal a bright royal blue spot where the antibody-PBS solution was placed, a less distinctive blue spot where the 31 pg/ml hapten-antibody solution was placed, and a light blue purple spot where the 10,000 pg/ml hapten was placed. The serial dilution should produce increasingly darker shades of blue as the hapten concentration decreased.
6. The physical representation of the color change is the change in the optical thickness coated to the surface of the wafer. This thickness can be measured by the comparison ellipsometer in gray scale units. The Table 3 below is a measurement of a different bound antibody. Because this data was generated by a similar hapten tested in a competitive format, it is believed that the results from the above given protocol would be comparative in nature.

**TABLE 3**

| Measurement of Bound Antibody | |
|---|---|
| Concentration Hapten ng/ml | Gray Scale Measurement |
| 0 pg/ml | 53.2 |
| 31 pg/ml | 40.42 |
| 62 pg/ml | 34.00 |
| 125 pg/ml | 27.39 |
| 500 pg/ml | 26.63 |
| 1,000 pg/ml | 23.27 |
| 10,000 pg/ml | 15.04 |

The less hapten present in the analyte solution the higher the concentration of available antibodies. The higher the concentration of available antibodies in the analyte solution the more surface binding between the immobilized hapten and the free solution antibody can occur. The more surface binding that occurs the more vibrant the interference color change. The reacted spots and the background interference color were nonspecularly reflected due to the irregular surface of the wafer.

### EXAMPLE 5

### Detection of Group A Streptococcus

Three silicon wafers with highly polished surfaces, and three silicon wafers with non-regular surfaces each having a refractive index of approximately 4 to 4.08 at a specified wavelength, were used in this test. Each group was coated with a similar anti-reflective material. Group A was coated with approximately 55 nm (550 Angstroms) of silicon monoxide having an index of refraction of 1.97 at a specified wavelength. Group B was coated with approximately 56 nm (560 Angstroms) of silicon oxynitride having an index of refraction at a specified wavelength of 1.95. Group C was coated with approximately 55 nm (550 Angstroms) of boron oxide having an index of refraction at a specified wavelength of 1.9. These coatings closely approximate the equation used for coating one optical layer to the surface of the substrate.

The receptive material was coated to a thickness of 2.5 to 3 nm (25 to 30 Angstroms). This figure was disregarded in calculating the optimal thickness of the anti-reflective material. The three groups of wafers were chemically activated to allow attachment of the receptive material. The activation was by application of N-2(2-aminoethyl)-3-aminopropyltrimethoxysilane by the following procedure:
1. The coated wafers were oxygen plasma etched for 5 minutes in a .70 Torr oxygen atmosphere with a plate current of 175 D.C. milliamperes and 250 RF watts.
2. Immediately upon removal from the oxygen plasma etcher the wafers were placed in a quartz rack which was inserted into a vacuum dessicator with an attached vessel into which 2.5 microliters of the aminosilane was placed.
3. The vacuum dessicator was evacuated to 0.06 Torr for 30 minutes. Then the temperature of the dessicator was raised to 100 degrees C over the course of one hour. This process activated the wafer by the vapor deposition of aminosilane onto the wafers' surfaces.
4. The wafers were now prepared for the adherence of the receptive materials. First, 250 micrograms per milliliter of Bovine Serum Albumin (BSA) was dissolved in Phosphate Buffered Saline (PBS). 10 milliliters of this solution was adjusted to pH 8.5 and was pipetted into a plastic culture cell box containing the six wafers.
5. 50 microliters of a 25% glutaraldehyde in water solution was added to the culture cell box. 25 microliters of a 50 micrograin per ml Protein A solution was also added to the culture cell box.
6. The six wafers in the culture cell box were incubated in a shaker bath at room temperature for one and a half hours.
7. 200 microliters of a commercially available (from Ventrex, Inc.) anti-Strep A (raised in rabbits) was pipetted into the culture cell box; and allowed to incubate for another hour in the shaker bath at room temperature.
8. Following incubation the culture cell box was refrigerated at 4 degrees C for approximately 48 hours to further adherence of the receptive antibody material to the device. To remove all unbound material the wafers were rinsed with distilled deionized water and dried in a stream of N₂.

The six wafers appeared shades of gold with three of the wafers providing a specular reflection and three of the wafers providing a non-specular reflection. These wafers were then used to determine the presence of Strep A in either a bacteria form or a bacteria lysate form.

An assay technique is described hereinafter:
1. Each wafer was separated and placed into a small plastic test kit. The test kit was designed with two rough grade filter paper pads attached to the inside of the top cover. Each test kit has seven microliters of a positive control placed on one pad and seven microliters of a negative control placed on the second pad. The positive control, commercially obtained from Becton Dickinson, was heat killed Group A streptococcus in solution with a 0.02% sodium azide. The negative control was taken from a solution of 50 micrograms of BSA per ml of PBS.
2. The cover of the test kits were closed placing the filter pads in contact with the coated wafer for one minute.
3. The covers were opened and the wafers were allowed to air dry for one minute. Then the wafers were rinsed with distilled deionized water and dried with pressurized air.

Visual inspection of the wafers revealed highly visible purple spots on the non-specular wafers and visible purple spots on the specular wafers. Visual inspection of the non-specular wafers demonstrated a lower angle dependence than that of the specular wafers. The reacted colors became less vibrant at lower concentrations, although all spots were clearly distinguishable. An instrument was used to verify the increase of mass produced by the binding reaction on the surface of the wafer. The change in mass or thickness was measured in gray scale units by a Sagax_{R} Comparison Ellipsometer.

The foregoing examples show various substrate materials, formats, anti-reflective materials, analytes, and receptive materials.

The design of these wafers can be used in a competitive or a non-competitive assay as depicted in Example 4 or as an inhibition assay. The assay can be an immunoassay or as depicted in Example 2, a clinical chemistry assay or an enzymatic assay or can be designed for detection of DNA or RNA. The substrate can be a variety of materials including metals such as chromium, see Example 3. When metals are used the imaginary refractive index has to be accounted for in the selected equations. This device can be produced as a wedged surface or a planar surface as either a qualitative or semi-quantitative device. The foregoing examples also show that the design can be a quantitative instrumented device. The optical layer of this device can be useful for both visual and instrumented detection. Instruments that are designed to read wavelengths or intensity of light can be used to read this wafer. Other instruments detecting various physical parameters can also be employed to make this wafer a qualitative or quantitative instrumented technique.

The foregoing examples serve to illustrate the efficiency and utility of this technology to detect a variety of analytes using the device consisting of a substrate, anti-reflective material(s), activation, and receptive material(s) to generate an interference color change or intensity change as a signal of analyte attachment.

Without being bound to the substrate formats or materials utilized in the preceding examples, it is possible to utilize a diversity of combinations of substrate formats and substrate materials which are functionally equivalent substitutes capable of having anti-reflective material bound to their surface.

The anti-reflective material(s) provides a layer(s) which can be activated to function as the receptive material or to attach or adhere, by whatever mechanism the receptive material to the apparatus. Furthermore, the anti-reflective materials utilized in this invention are coated to approximately a quarter wavelength optical thickness of a designated wavelength of light to achieve the highest level of destructive light interference.

The AR coating can vary from a quarter of the designated wavelength, namely because destructive interference need not be total to achieve an adequate interference color, and because both polychromatic and monochromatic light can be employed to irradiate the device. The actual amount of AR material coated to the surface can vary widely because the designated wavelength can be almost any wavelength and because increasing the thickness of the AR coating yields a device which is increasingly sensitive to the binding of even small analytes.

The material composition of the AR layers are selected based on the refractive index of the AR materials and the substrate materials selected; therefore almost any AR material can be utilized if the correct combination of materials is selected. The top AR material is selected to be capable of being activated to function as, or to receive, the receptive material. Most AR materials can be activated by some means to allow attachment of receptive material to the apparatus; therefore a multitude of materials are capable of being utilized as anti-reflective materials.

Various methods of chemical activation can be utilized dependent on the composition of the AR material and the receptive material, or if the activation material(s) are acting as the receptive material then dependent on the composition of the AR material(s) and the analyte. A variety of materials, functional groups, etc. can function as the activation material or as the activation process.

The substrate, which can be any of a variety of shapes, i.e. test tubes, wafers, glass slides, microwells, etc., and formats, i.e. a solid support, a rigid flexible or semi-flexible support, a gel, a pellicle and made of various suitable materials i.e., glass, silicon, plastics such as polymeric hydrocarbons like polystyrene, etc. and made of materials which are either nonspecularly or reflective, or transmissive, specularly, can be treated with the aforementioned technology affording many useful approaches to the detection of the analyte.

This analyte detection need not be limited to visual detection of an interference color change. The color change can be in the infrared or the ultra-violet regions wherein the analyte detection is pursuant to an instrumented detection apparatus. The reacted apparatus can be qualitatively or quantitatively analyzed by an ellipsometer, or any other instrument capable of detecting by whatever mechanism the analyte binding.

## Claims

1. An analyte detecting article comprising: a substrate supporting on its surface not less than one layer of anti-reflective material adhering thereto;
the top layer of said anti-reflective material supporting on its surface a layer of analyte receiving material capable of analyte interaction;
said anti-reflective layer comprising an anti-reflective coating having a thickness of about one 1/4 the wavelength of light in the medium and a refractive index of about the square root of the product of the indices of refraction of the medium directly above and below said layer.

2. The article of claim 1 wherein said substrate is a gel, a pellicle, a flexible material, is transmissive, reflective or is chromium.

3. The article in accordance with claim 1 wherein said anti-reflective material is coated on the substrate as a patterned or a wedge shaped design.

4. An analyte detecting article in accordance with claim 1 where the analyte detected is a tumor marker, a carcinoembryonic antigen, an autoimmune indicator, a rheumatoid factor, an amine binding factor present in the urine of pregnant women, a hapten, a bacteria, a bacteria lysate, or Streptococcus A.

5. The article of claim 1 comprised of two discrete layers consisting of one layer of activating material and one layer of anti-reflective material.

6. The article of claim 1 having a top layer of anti-reflective material capable of being activated to capture an analyte from a test solution.

7. The article in accordance with claim 1 wherein said substrate is silicon.

8. The article in accordance with claim 1 wherein said anti-reflective material is silicon oxynitride or boron oxide.

9. The article in accordance with claim 1 wherein the substrate is metal or non-metal, a glass, plastic or a polymer.

10. A process for selectively detecting the presence of an analyte in solution which comprises the steps of:
depositing one or more layers of anti-reflective material on a substrate, said one or more layers of anti-reflective material comprising an anti-reflective coating having a thickness of about one 1/4 the wavelength of light in the medium and a refractive index of about the square root of the product of the indices of refraction of the medium directly above and below said layer,
contacting a coated support article with a solution to be tested for the presence of said analyte in the solution, such that the analyte binds to the article causing a change in reflection of said article,
detecting the analyte attachment by detecting the change in the reflection of said article.

11. The process of claim 10 wherein detecting the presence of the analyte is performed by instrumentation, or by human vision, or with at least one filter or with at least one polarizer or at an angle other than normal.

12. The process of claim 11 including the step of irradiating the analyte detecting article with light which is monochromatic, polychromatic, ultraviolet or infrared.

13. The process of claim 10, wherein the top layer of said anti-reflective material supports on its surface a layer of analyte receiving material capable of analyte interaction.

## Patentansprüche

1. Artikel zum Nachweis eines Analyten, umfassend:
Substrat, welches auf seiner Oberfläche nicht weniger als eine Schicht eines daran haftenden anti-reflektiven Materials trägt;
wobei die Oberseitenschicht des anti-reflektiven Materials auf ihrer Oberfläche eine Schicht eines Analyten-aufnahmefähigen Materials trägt, welches fähig zur Wechselwirkung mit einem Analyten ist;
wobei die anti-reflektive Schicht eine anti-reflektive Beschichtung umfaßt, welche eine Dicke von etwa einem ¼ der Wellenlänge des Lichts in dem Medium und einen Brechungsindex von etwa der Quadratwurzel des Produkts der Brechungsindizes des Mediums direkt überhalb und unterhalb der Schicht besitzt.

2. Artikel von Anspruch 1, worin das Substrat ein Gel, ein Häutchen, ein flexibles Material ist, lichtdurchlässig, reflektiv oder Chrom ist.

3. Artikel gemäß Anspruch 1, worin das anti-reflektive Material auf dem Substrat in einer gemusterten oder keilförmigen Gestaltung aufbeschichtet ist.

4. Artikel zum Nachweis eines Analyten gemäß Anspruch 1, wobei der nachgewiesene Analyt ein Tumormarker, ein karzinoembryonisches Antigen, ein Autoimmun-Indikator, ein Rheumatoid-Faktor, ein im Urin schwangerer Frauen vorhandener Amin-Bindungsfaktor, ein Hapten, ein Bakterium, ein Bakterienlysat oder Streptococcus A ist.

5. Artikel von Anspruch 1, aufgebaut aus zwei getrennten Schichten, bestehend aus einer Schicht aus aktivierendem Material und einer Schicht aus anti-reflektivem Material.

6. Artikel von Anspruch 1 mit einer Oberseitenschicht aus anti-reflektivem Material, welche fähig ist, zum Einfangen eines Analyten aus einer Testlösung aktiviert zu werden.

7. Artikel gemäß Anspruch 1, worin das Substrat Silicium ist.

8. Artikel gemäß Anspruch 1, worin das anti-reflektive Material Siliciumoxynitrid oder Boroxid ist.

9. Artikel gemäß Anspruch 1, worin das Substrat ein Metall oder Nichtmetall, ein Glas, Kunststoff oder ein Polymer ist.

10. Verfahren zum selektiven Nachweis der Gegenwart eines Analyten in Lösung, welches die folgenden Schritte umfaßt:
Auftragen einer oder mehrerer Schichten aus anti-reflektivem Material auf einem Substrat, wobei die eine oder die mehreren Schichten aus anti-reflektivem Material eine anti-reflektive Beschichtung umfassen, welche eine Dicke von etwa einem ¼ der Wellenlänge des Lichts in dem Medium und einen Brechungsindex von etwa der Quadratwurzel des Produkts der Brechungsindizes des Mediums direkt überhalb und unterhalb der Schicht besitzt,
Kontaktieren eines beschichteten Trägerartikels mit einer hinsichtlich der Gegenwart des Analyten in der Lösung zu testenden Lösung, so daß der Analyt an den Artikel bindet, was eine Veränderung in der Reflexion des Artikels verursacht,
Nachweisen der Analytenanlagerung durch Nachweis der Veränderung in der Reflexion des Artikels.

11. Verfahren von Anspruch 10, worin das Nachweisen der Gegenwart des Analyten durch Instrumente oder durch menschliches Sehvermögen oder mit mindestens einem Filter oder mit mindestens einem Polarisator oder bei einem Winkel, der von der Normalen verschieden ist, durchgeführt wird.

12. Verfahren von Anspruch 11, worin der Schritt des Bestrahlens des Artikels zum Nachweis eines Analyten mit Licht eingeschlossen ist, welches monochromatisch, polychromatisch, ultraviolett oder infrarot ist.

13. Verfahren von Anspruch 10, worin die Oberseitenschicht des anti-reflektiven Materials auf ihrer Oberfläche eine Schicht eines Analyten-aufnahmefähigen Materials trägt, welches fähig zur Wechselwirkung mit einem Analyten ist.

## Revendications

1. Article détectant un analyte, comprenant : un substrat supportant sur sa surface pas moins d'une couche de matière anti-réfléchissante adhérant à celle-ci ;
la couche supérieure de ladite matière anti-réfléchissante supportant sur sa surface une couche de matière recevant un analyte, capable d'intéraction avec l'analyte ;
ladite couche anti-réfléchissante comprenant un revêtement anti-réfléchissant ayant une épaisseur d'environ un 1/4 de la longueur d'onde de lumière dans le milieu et un indice de réfraction d'environ la racine carrée du produit des indices de réfraction du milieu directement au-dessus et en dessous de ladite couche.

2. Article selon la revendication 1, dans lequel ledit substrat est un gel, une pellicule, une matière flexible, est transmittif, réfléchissant ou est du chrome.

3. Article selon la revendication 1, dans lequel ladite matière anti-réfléchissante est déposée sous forme d'un revêtement sur le substrat selon un motif ou en biseau.

4. Article détectant un analyte selon la revendication 1 où l'analyte détecté est un marqueur de tumeur, un antigène carbino-embryonnaire, un indicateur auto-immun, un facteur rhumatoïde, un facteur de liaison d'amine présent dans l'urine de la femme enceinte, un haptène, une bactérie, un lysat de bactérie ou un streptocoque A.

5. Article selon la revendication 1, constitué de deux couches discrètes consistant en une couche de matière activante et une couche de matière anti-réfléchissante.

6. Article selon la revendication 1, ayant une couche du dessus de matière anti-réfléchissante capable d'être activée pour capturer un analyte à partir d'une solution d'essai.

7. Article selon la revendication 1, dans lequel ledit substrat est du silicium.

8. Article selon la revendication 1, dans lequel ladite matière anti-réfléchissante est de l'oxynitrure de silicium ou de l'oxyde de bore.

9. Article selon la revendication 1, dans lequel le substrat est du métal ou non-métal, un verre, du plastique ou un polymère.

10. Procédé pour détecter sélectivement la présence d'un analyte en solution, qui comprend les étapes consistant à :
déposer une ou plusieurs couches de matière anti-réfléchissante sur un substrat, lesdites une ou plusieurs couches de matière anti-réfléchissante comprenant un revêtement anti-réfléchissant ayant une épaisseur d'environ un 1/4 de la longueur d'onde de lumière dans le milieu et un indice de réfraction d'environ la racine carrée du produit des indices de réfraction du milieu directement au-dessus et en dessous de ladite couche,
mettre en contact un article support revêtu, avec une solution à tester quant à la présence dudit analyte dans la solution, de sorte que l'analyte se lie à l'article en provoquant un changement de réflexion dudit article,
détecter l'attachement de l'analyte en détectant le changement dans la réflexion dudit article.

11. Procédé selon la revendication 10, dans lequel la détection de la présence de l'analyte est effectuée par instrumentation, ou par vision humaine, ou avec au moins un filtre ou avec au moins un polariseur ou à un angle autre que normal.

12. Procédé selon la revendication 11, incluant l'étape consistant à irradier l'article détectant un analyte avec une lumière qui est monochromatique, polychromatique, ultraviolette ou infrarouge.

13. Procédé selon la revendication 10, dans lequel la couche du dessus de ladite matière anti-réfléchissante supporte sur sa surface une couche de matière recevant un analyte capable d'intéraction avec l'analyte.
